# FASCICULE DE BREVET EUROPEEN

(11) **EP 0 956 005 B1**
(45) Date de publication et mention de la délivrance du brevet: **19.01.2005**
(21) Numéro de dépôt: 97940223.7
(22) Date de dépôt: 11.09.1997
(51) Int. Cl.: A61K 9/48, A61K 9/00

(54) **DISPOSITIF ET PROCEDE DE FABRICATION D'UNE CAPSULE TUBULAIRE SEGMENTEE CONTENANT UN MILIEU BIOLOGIQUEMENT ACTIF**
VORRICHTUNG UND VERFAHREN ZUR HERSTELLUNG VON UNTERTEILTEN ROHRFÖRMIGEN KAPSELN MIT EINER BIOLOGISCH AKTIVEN ZUSAMMENSETZUNG
DEVICE AND METHOD FOR MAKING A SEGMENTED TUBULAR CAPSULE CONTAINING A BIOLOGICALLY ACTIVE MEDIUM

(30) Priorité: 12.09.1996 FR 9611346
(43) Date de publication de la demande: 17.11.1999
(73) Titulaire: HOSPAL INDUSTRIE S.A., F-69883 Meyzieu Cédex (FR)
(72) Inventeur: MUSCAT, Eric, F-69100 Villeurbanne (FR); BRUN, Claude, F-69008 Lyon (FR)
(74) Mandataire: Sutto, Luca
(86) Numéro de dépôt international: PCT/FR1997/001609
(87) Numéro de publication internationale: WO 1998/010755

(56) Documents cités:
- EP-A- 0 200 224
- WO-A-91/10425
- JIRI HONIGER; ET AL.: "Permeability and biocompatibility of a new hydrogel used for encapsulation of hepatocytes" BIOMATERIALS, vol. 16, no. 10, 1995, GB, pages 753-759, XP000673381

## Description

La présente invention concerne la fabrication d'une capsule contenant un milieu biologiquement actif, destinée à être implantée dans un organisme vivant à des fins thérapeutiques. De façon classique, une capsule de ce type est constituée d'un noyau comprenant un milieu biologiquement actif, qui est entouré d'une enveloppe semi-perméable. Le rôle de l'enveloppe est d'isoler le milieu biologiquement actif des tissus de l'organisme receveur tout en autorisant le passage, au travers de l'enveloppe, des substances par lesquelles la capsule implantée remplit ses fonctions. A titre d'exemple, lorsque le milieu biologiquement actif est une suspension de cellules, l'enveloppe semi-perméable doit faire obstacle aux réactions immunitaires, doit permettre la diffusion de substances nutritives vers le noyau de la capsule et la diffusion, vers l'organisme, de la substance d'intérêt thérapeutique sécrétée par les cellules (insuline, par exemple lorsque les cellules sont des îlots de Langerhans).

Du point de vue de l'efficacité des échanges au travers de la membrane, il semble que les capsules sphériques soient préférables aux capsules tubulaires. Les capsules sphériques présentent cependant l'inconvénient d'être difficilement localisables lorsqu'elles sont implantées, de sorte qu'il semble exclu aujourd'hui qu'on puisse les implanter dans un corps humain d'où les implants doivent toujours pouvoir être extraits pour des raisons de sécurité biologique.

Les recherches récentes se sont donc principalement orientées vers la fabrication de capsules tubulaires, qui permettent la mise en forme d'implants suffisamment longs pour être repérables aisément. II a été découvert notamment qu'il est préférable de segmenter les capsules tubulaires, c'est-à-dire d'y ménager des compartiments isolés les uns des autres, de façon que, si une partie d'une capsule implantée est endommagée, il soit possible de la séparer de l'implant et de l'extraire de l'organisme receveur.

Le brevet US 5 158 881 décrit un procédé de fabrication d'une capsule tubulaire segmentée comprenant les étapes de :
- coextruder une solution. de polymère destinée à former l'enveloppe de la capsule et un milieu biologiquement actif, par injection simultanée de la solution de polymère et du milieu biologiquement actif dans une filière, et
- interrompre à intervalles de temps déterminés l'injection du milieu biologiquement actif pour ménager dans la fibre des compartiments successifs remplis par le milieu biologiquement actif et séparés par un tronçon intercalaire plein constitué uniquement de la solution de polymère.

Ce procédé ne produit pas les résultats escomptés lorsque la solution de polymère est liquide car, lorsque l'injection du milieu biologiquement actif est interrompue, il se forme, sous la filière une goutte de solution de polymère qui se déforme et se rompt sous l'effet du poids de la fibre déjà formée qui pend de la filière.

L'invention a pour but d'améliorer le procédé décrit ci-dessus de façon à rendre possible la fabrication d'une capsule tubulaire segmentée à partir d'une solution de polymère liquide. L'invention a également pour but la mise en forme d'une capsule tubulaire sous une forme directement implantable.

Pour atteindre ce but, on prévoit, selon l'invention, un procédé de fabrication d'une capsule, tubulaire comprenant une paroi délimitant des compartiments internes isolés les uns des autres, la paroi étant faite à partir d'une solution d'au moins un polymère et les compartiments étant remplis d'un milieu biologiquement actif, le procédé comprenant les étapes de:
- coextruder la solution d'au moins un polymère et le milieu biologiquement actif par injection simultanée de la solution de polymère et du milieu biologiquement actif dans une filière ayant des dimensions déterminées,
- interrompre à intervalles de temps déterminés l'injection du milieu biologiquement actif pour ménager dans la fibre des compartiments successifs remplis par le milieu biologiquement actif et séparés par un tronçon intercalaire plein constitué uniquement de la solution de polymère,
- achever la coagulation de la fibre (11), le procédé étant caractérisé en ce qu'il comporte l'étape de:
   - provoquer ou sortir de la filière et avant l'achèvement de la coagulation, une solidification précoce partielle de la fibre (11) suffisante pour prévenir la rupture de la fibre au niveau des tronçons intercalaires.

De préférence, l'étape de solidification précoce partielle de la fibre comprend les étapes de:
- immerger la fibre dans un liquide de coagulation au sortir de la filière de façon à initier une coagulation précoce de la solution de polymère par l'extérieur de la fibre; et
- entraîner simultanément la fibre dans le liquide de coagulation selon un trajet déterminé.

Ce procédé présente au moins deux intérêts majeurs. D'une part, il permet d'envisager une production industrielle ou semi-industrielle d'implants. D'autre part, la mise en contact précoce de la fibre sortant de la filière avec le liquide de coagulation permet l'extraction d'une partie du solvant utilisé pour préparer la solution de polymère, ce qui est particulièrement souhaitable lorsque le milieu biologiquement actif comprend des cellules vivantes qui peuvent être affectées par les solvants couramment utilisés.

Selon une caractéristique de l'invention, le procédé comporte, simultanément à l'étape de solidification partielle de la fibre, l'étape d'exercer une force de traction déterminée sur la fibre de façon à lui conférer au moins une caractéristique géométrique indépendante des dimensions de la filière.

Grâce à cette disposition, il est possible de filer des solutions de polymère de faible viscosité.

Selon une autre caractéristique de l'invention, le procédé comporte en outre l'étape de donner à la fibre une forme permanente. Par exemple, la fibre est enroulée sur un mandrin cylindrique de façon à prendre la forme d'une spirale dont le pas est choisi pour que chaque section de fibre comprenant un nombre entier de compartiments correspondant à une capsule implantable, occupe une longueur de mandrin déterminée.

Grâce à cette disposition, les dimensions de la capsule implantable peuvent être ajustées selon la taille de l'organisme receveur. On a observé que la forme spiralée était particulièrement adaptée aux implants en ce qu'elle leur confère une tenue mécanique, en ce qu'elle en rend aisée la manipulation et en ce qu'elle en facilite l'implantation dans un organisme.

L'invention a aussi pour objet un dispositif de fabrication d'une fibre tubulaire comprenant une paroi délimitant des compartiments internes isolés les uns des autres et remplis d'un milieu biologiquement actif, comprenant:
- des moyens de filage ayant des dimensions déterminées pour coextruder une solution d'au moins un polymère et le milieu biologiquement actif de façon à obtenir une fibre tubulaire ayant une paroi faite à partir de la solution de polymère et remplie du milieu. biologiquement actif;
- des moyens d'alimentation des moyens de filage avec la solution de polymère et avec le milieu biologiquement actif;
- des moyens pour commander l'alimentation simultanée des moyens de filage en solution de polymère et en milieu biologiquement actif et pour interrompre l'alimentation en milieu biologiquement actif à intervalles de temps déterminés de façon à ménager dans la fibre des compartiments successifs remplis par le milieu biologiquement actif et séparés par un tronçon intercalaire plein constitué uniquement par la solution de polymère;
- des moyens (14) pour achever la coagulation de la fibre (11); le dispositif étant caractérise en ce qu'il comporte:
   - des moyens (10) pour provoquer une solidification précoce partielle de la fibre au sortir des moyens de filage (7) cette solidification étant suffisante pour prévenir la rupture de la fibre (11) au niveau des tronçons intercalaires (11a),
   - lesdits moyens (10) étant placés en amont des moyens (14) dans le sens de déplacement de la fibre.

De préférence, les moyens de solidification comprennent:
- un bac pour un liquide de coagulation disposé sous les moyens de filage à une distance choisie pour qu'une fibre s'écoulant de la filière à un débit donné commence à coaguler précocement de l'extérieur et ne se rompe pas; et
- des moyens pour entraîner la fibre dans le liquide de coagulation selon un trajet déterminé.

Selon une caractéristique de l'invention, le dispositif comprend en outre des moyens pour exercer une force de traction déterminée sur la fibre de façon à lui conférer au moins une caractéristique géométrique indépendante des dimensions des moyens de filage.

Dans un mode de réalisation de l'invention, les moyens d'entraînement et les moyens pour exercer une force de traction comprennent un tube traversant le fond du bac de coagulation et ayant une extrémité supérieure à l'intérieur du bac et une extrémité inférieure à l'extérieur du bac, ce tube étant disposé sensiblement à la verticale et son extrémité supérieure étant située au dessous de la surface du liquide de coagulation lorsque le bac est rempli à un niveau déterminé de fonctionnement.

Ce mode de réalisation est particulièrement avantageux puisque, avec des moyens techniques très simples et peu coûteux il permet de remplir plusieurs fonctions. Par ailleurs, il est facile à régler et permet d'exercer sur la fibre, le cas échéant, des tractions très faibles.

Selon une autre caractéristique de l'invention, le dispositif comprend en outre des moyens pour donner à la fibre une forme déterminée. Par exemple, la forme choisie est une spirale et les moyens pour donner cette forme à la fibre comprennent:
- un mandrin constitué de portions démontables;
- des moyens pour entraîner le mandrin en rotation;
- un dispositif de trancanage pour déplacer la fibre selon un mouvement de va-et-vient parallèlement au mandrin.

Avantageusement, en fonctionnement, la vitesse de rotation du mandrin et la vitesse du mouvement de va-et-vient du dispositif de trancanage sont choisies pour que la section de fibre enroulée en spirale sur la longueur d'une portion de mandrin corresponde approximativement à un nombre entier déterminé de compartiments internes de la fibre.

Grâce à cette disposition, il est possible de limiter au maximum la manipulation de la fibre mise en forme : il suffit de prévoir que, sur chaque portion de mandrin, on enroule la longueur de fibre nécessaire pour constituer une capsule implantable. Lorsque de la fibre est enroulée sur toute la longueur du mandrin, on coupe la fibre à la jonction entre deux portions de mandrins contiguës et on désolidarise les portions de mandrins qui peuvent servir de support aux capsules implantables pour leur expédition et leur stockage.

D'autres caractéristiques et avantages de l'invention apparaîtront à la lecture de la description qui suit. On se reportera aux dessins annexés sur lesquels:
La figure 1 représente, de façon schématique, une vue en plan d'une première partie d'un dispositif pour la fabrication d'une capsule tubulaire segmentée selon l'invention ;
La figure 2 représente, de façon schématique, les moyens de filage et de solidification du dispositif selon l'invention ;
La figure 3 représente de façon schématique, une vue de dessus d'une deuxième partie d'un dispositif pour la fabrication d'une capsule tubulaire segmentée selon l'invention.

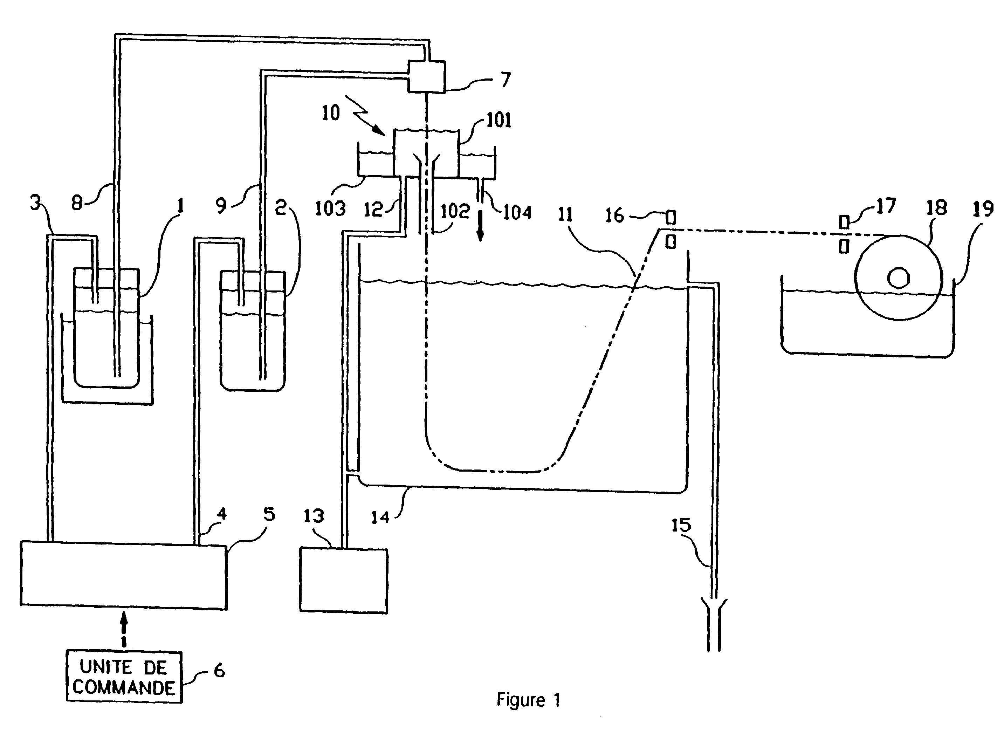

Le dispositif de la figure 1 comprend un premier réservoir 1 pour contenir un milieu biologiquement actif, sous forme liquide, tel qu'une suspension de cellules. Ce réservoir est placé dans une enceinte de régulation de température permettant de maintenir le contenu du réservoir 1 à température constante. Un second réservoir 2 est prévu pour contenir une solution de polymère. Les deux réservoirs 1 et 2 sont hermétiquement fermés et sont reliés par des canalisations 3, 4 à un système de régulation de pression gazeuse 5 permettant d'ajuster et de maintenir une pression constante dans chaque réservoir. Le système de régulation de pression 5 est connecté à une unité de commande 6 qui commande la régulation de la pression dans chaque réservoir en fonction de valeurs de consignes préalablement communiquées par un opérateur à l'unité de commande 6 au moyen d'un clavier (non représenté).

Les réservoirs 1 et 2 et le système 5 de régulation de pression gazeuse constituent les moyens d'alimentation de moyens de filage 7 ayant deux buses tubulaires concentriques, la buse intérieure 71 étant reliée par une canalisation 8 au réservoir 1 et la buse extérieure 72 étant reliée par une canalisation 9 au réservoir 3.

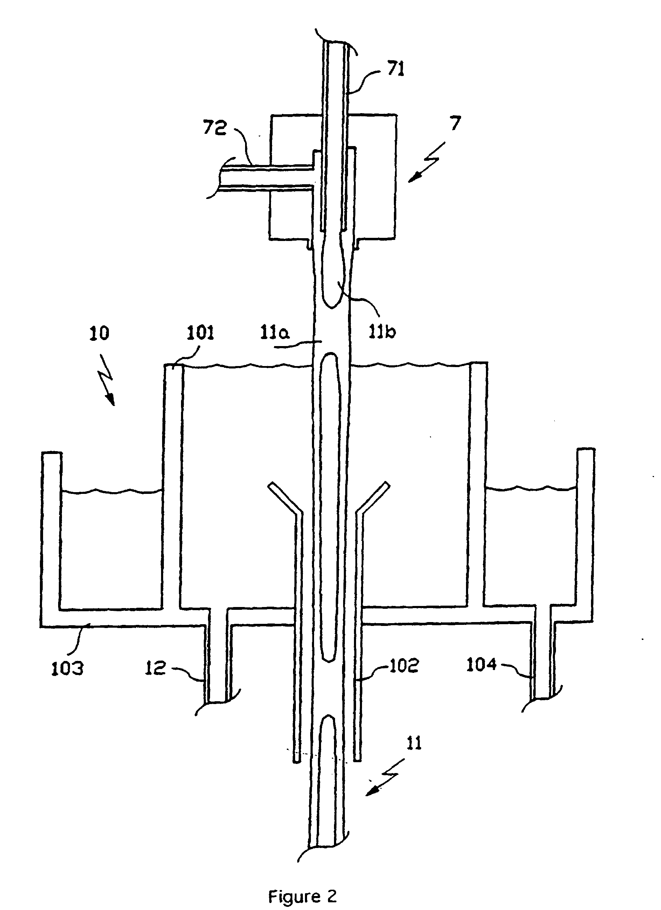

Conformément à l'invention, des moyens 10 pour provoquer une solidification précoce d'une fibre tubulaire 11 issue des moyens de filage sont disposés à l'aplomb des buses concentriques 71, 72. Comme on peut le voir plus en détail sur la figure 2, ces moyens de solidification précoce comprennent un bac de coagulation 101 ayant un fond percé d'un trou pour le passage d'un tube 102, évasé à sa partie supérieure, dont l'axe longitudinal central est sensiblement aligné avec l'axe longitudinal central des buses 71, 72. Par suite de cette disposition, le tube 102 a une extrémité supérieure à l'intérieur du bac de coagulation 101 et une extrémité inférieure à l'extérieur de ce bac. Le tube 102 est monté pour pouvoir coulisser selon une direction verticale, de sorte que la hauteur d'eau entre l'extrémité supérieure du tube 102 et un niveau d'eau de référence dans le bac de coagulation 102 peut être ajustée avec précision.

Le choix de la longueur du tube 102, de son diamètre interne, et de la hauteur d'eau entre le niveau de référence et l'extrémité supérieure du tube permet de régler précisément l'action exercée sur la fibre, simple entraînement ou traction. Pour une même hauteur d'eau, et pour un tube de même diamètre interne, plus le tube est long, moindre est la traction. Pour une même hauteur d'eau, et pour un tube de même longueur, plus le diamètre interne du tube est faible, moindre est la traction. Pour un tube de même longueur et de même diamètre interne, moins la hauteur d'eau est importante, moindre est la traction.

Dans le mode de réalisation représenté, le niveau d'eau de référence, par rapport auquel la position du tube 102 dans le bac est réglé, est défini par le remplissage du bac 101 à ras bord. Afin de maintenir constamment le niveau de référence et de renouveler en permanence le liquide de coagulation dans le bac 101, le bac 101 est placé dans une cuve de trop plein 103 ayant un orifice de trop plein 104. Le bac de coagulation 10 est relié par une canalisation 12 à une source 13 de liquide de coagulation, qui, en fonctionnement, alimente le bac de coagulation 101 en continu.

Un second bac de coagulation 14 est disposé à l'aplomb du tube 102 pour recevoir la fibre 11 et en achever la coagulation. La distance entre l'extrémité inférieure du tube 102 et le niveau de liquide dans le second bac de coagulation 14 est ajustée selon la traction souhaitée sur la fibre au sortir du tube 102. Plus cette distance est grande, plus forte est la traction, laquelle est causée par la gravité. Accessoirement, le second bac de coagulation 14 est utilisé pour recueillir le liquide de la cuve de trop plein 103 du premier bac de coagulation 101. Le second bac de coagulation 14 est lui aussi relié à la source 13 de liquide de coagulation et il est muni d'une canalisation de trop plein 15 permettant le renouvellement continu de son contenu.

Le dispositif comporte des organes de guidage 16, 17 pour guider la fibre hors du second bac de coagulation 14 vers un cylindre 18 rotatif destiné au stockage temporaire de la fibre coagulée. Le cylindre 18 est disposé dans un bac de lavage 19.

La température du liquide de coagulation dans les bacs 101 et 14 de même que la température du liquide de lavage dans le bac 19 peut être ajustée à toute valeur de consigne grâce à des moyens de régulation de température (non représentés).

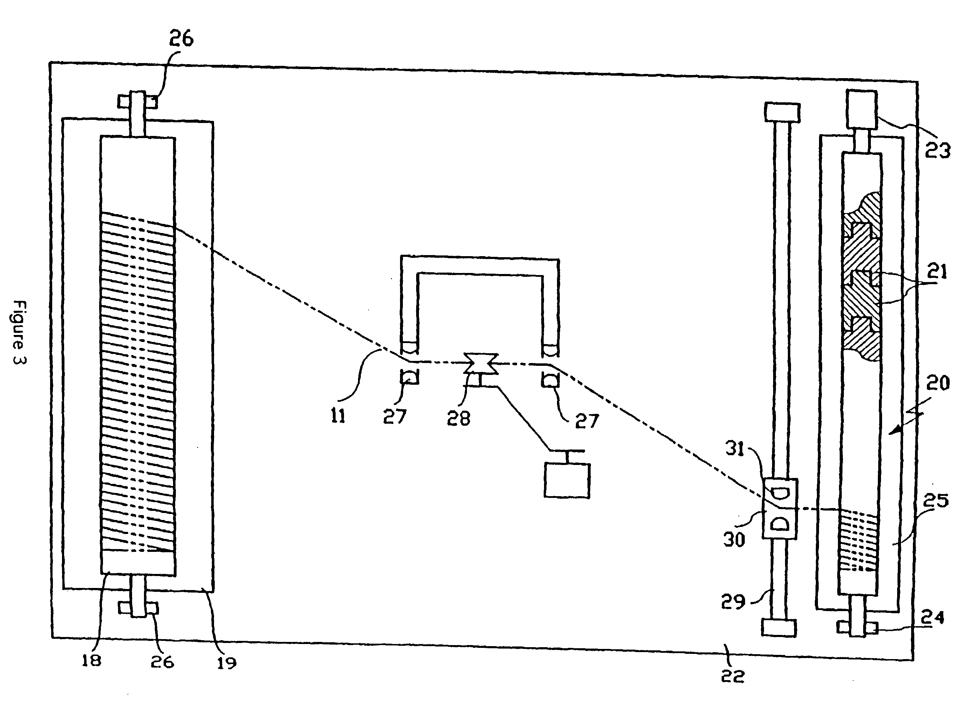

Conformément à l'invention, le dispositif comprend aussi des moyens de mise en forme de la fibre tubulaire segmentée. On a remarqué qu'avec certaines solutions de polymère, si, dans une période de temps déterminée après que la fibre a été retirée du bain de coagulation, on soumet la fibre à une déformation mécanique pendant un temps donné et à une température donnée, cette déformation devient permanente. Dans le mode de réalisation représenté sur la figure 3, les moyens de mise en forme comprennent un mandrin cylindrique 20 constitué de portions emboîtables 21. Ce mandrin est monté de façon amovible sur un bâti 22 de façon à être immergé partiellement dans un deuxième bac de lavage 25. Une de ses extrémités est couplée à un moteur rotatif 23 et son autre extrémité est supportée par un palier 24 est et libre en rotation. Le bâti 22 comprend en outre des moyens de support (paliers 26) pour recevoir le cylindre de stockage 18, de façon que celui-ci soit mobile en rotation autour de son axe longitudinal et soit disposé parallèlement au mandrin 20. Entre le cylindre de stockage 18 et le mandrin 20, divers organes mécaniques sont assujettis au bâti 22 pour permettre de transférer sur le mandrin 20 une partie de la fibre enroulée sur le cylindre 18. Ces organes mécaniques comprennent: - deux guides 26, 27 permettant de maintenir la fibre 11 perpendiculaire au cylindre 18 et au mandrin 20 sur une partie de sa longueur; - un organe de tension 28 exerçant une poussée verticale sur la fibre 11 entre les deux guides 26, 27; - un dispositif de trancanage comprenant un rail 29 parallèle au mandrin 20 et un chariot 30 mobile sur le rail 29 selon un mouvement de va-et-vient. Le chariot mobile est muni d'un guide 31 pour la fibre 11.

Le dispositif qui vient d'être décrit fonctionne de la façon suivante. L'ensemble du dispositif est placé dans une hotte à flux laminaire et tous les liquides utilisés (coagulation, lavage) sont stériles. Le contenu des bacs de coagulation 101, 14 et de lavage 19 est maintenu à température constante. Les réservoirs 1, 2 sont remplis pour l'un de solution de polymère, pour l'autre d'une suspension de cellules à encapsuler. L'enceinte de régulation de température est réglée pour maintenir la suspension de cellule à une température appropriée. Les divers bacs 101, 14, 19 sont remplis. La valeur de la pression de gaz pour chacun des réservoirs 1, 2 est communiquée à l'unité de commande 6. Ce sont ces valeurs qui déterminent en particulier le débit d'écoulement de la fibre tubulain 11 hors de la filière 7. Lorsque la mise en route du dispositif es achevée, l'unité de commande 6 pilote le système de régulation de pression de gaz 5 de façon que l'alimentation de la filière 7 avec la suspension de cellules soit interrompue à intervalles de temps réguliers. La fibre qui sort de la filière est alors un jonc plein 11a constitué uniquement de solution de polymère. Selon l'invention, pou prévenir la rupture de la fibre 11 au niveau de ce jonc plein 11a, qu est une goutte de liquide, on provoque une solidification précoce de la fibre en l'immergeant, à faible distance de sa sortie de la filière 7 dans un liquide de coagulation tout en l'entraînant à la verticale vers le bas au moyen du tube 102 immergé. On a remarqué qu'en l'absence d'entraînement, la fibre soumise à la force d'Archimède dans le liquide de coagulation se déforme longitudinalement en formant des zigzags et présente une section très irrégulière, ce qui la rend inutilisable comme implant. Comme mentionné plus haut, on peut aussi ajuster la hauteur du tube 102 dans le bac 101 de façon à créer une force de traction sur la fibre 11 permettant de régler les dimensions de la fibre (diamètre extérieur, épaisseur de paroi) indépendamment des dimensions des buses 71, 72 de la filière 7. Dès que la fibre 11 pénètre dans le liquide de coagulation, l'échange solvant/non solvant (qui définit la coagulation) se produit de l'extérieur de la fibre et vient limiter les effets indésirables d'une coagulation purement interne qui commence dès que la suspension de cellules entre au contact de la solution de polymère. En d'autres termes, la quantité de solvant (plus ou moins toxique pour les cellules) qui est extraite de la fibre par l'extérieur diminue d'autant la quantité de solvant susceptible de migrer dans la suspension de cellules lors du processus de coagulation par l'intérieur.

La fibre 11 qui sort du tube 102 est reçue dans le second bac 14 de coagulation où elle se solidifie suffisamment pour être manipulable. On note que le liquide de coagulation est renouvelé en permanence dans les bacs 101 et 14 pour éliminer le solvant. La fibre est ensuite lavée et enroulée sur le cylindre de stockage 18 pour former une seule spirale commençant et finissant respectivement à chaque extrémité du cylindre.

Pour la mise en forme de la fibre, un mandrin 20 est monté sur le banc 22. Une extrémité de la fibre enroulée sur le cylindre 18 est passée dans les guides 26, 27, puis dans le guide 31 du chariot 30 du dispositif de trancanage et elle est assujettie à une extrémité du mandrin 20. La longueur des portions 21 démontables du mandrin 20, de même que leur diamètre, sont prévus pour la mise en forme et le stockage définitif de capsules implantables comprenant un nombre déterminé de compartiments 11b. La vitesse du moteur 23 entraînant le mandrin 20 en rotation, ainsi que la vitesse linéaire du chariot 30 du dispositif de trancanage sont choisies de façon que, sur chaque portion 21 du mandrin 20, une section de fibre comprenant un nombre entier de compartiments 11b soit enroulée et qu'à chaque extrémité de chaque portion de mandrin 21 corresponde une section de fibre formée d'un jonc plein 11 a. La vitesse de rotation du mandrin 20 est augmentée chaque fois que la fibre arrive au niveau de l'extrémité de chaque portion de mandrin 21 de façon que les spires formées de jonc plein soient plus distantes les unes des autres que le reste des spires et que la séparation de deux capsules implantables adjacentes soit facilitée. Une seule spirale est formée sur le mandrin 20 commençant et finissant respectivement à chaque extrémité du madrin. On peut ensuite séparer les capsules implantables les unes des autres tout en les maintenant assujetties pour leur stockage et leur transport à la portion du mandrin sur laquelle elles ont été respectivement spiralées.

### Exemple 1:

Le réservoir 2 a été rempli d'une solution de polymère comprenant 8 % en poids d'un copotymère d'acrylonitrile et de méthallyle sulfonate de sodium (connu sous le nom commercial AN69), 6 % en poids de sérum physiologique (solution de chlorure de sodium dans de l'eau, à une concentration de 9 g/l) et 86 % de diméthylsulfoxide (DMSO). Le contenu du réservoir est à température ambiante (environ 25°C).

Le réservoir 1 a été rempli d'une suspension d'îlots de Langerhans à 10 000 IE/ml (IE = îlot-équivalent, correspondant à un îlot théorique de 150 µm de diamètre) dans de l'agarose (Sigma Type IA-A 0169 - N° lot - 54 H 0530) à 0,5% (poids/volume) résultant du mélange d'une suspension d'îlots dans du HAM'S F12 (Sigma N8641-N° lot - 123 H 2322) et d'une solution d'agarose à 0,65 % (poids/volume) dans du sérum physiologique. Le contenu du réservoir 1 est maintenu à 40,5°C .

Les bacs de coagulation 101 et 14 sont remplis de liquide physiologique stérile qui est renouvelé en permanence. La filière utilisée, fabriquée par la société SCP-France, a les dimensions suivantes : diamètre intérieur de la buse externe 72 = 1570 µm ; diamètre extérieur de la buse interne 71 = 980 µm ; diamètre intérieur de la buse interne 71 = 860 µm.

Le tube 102 des moyens pour provoquer la solidification précoce de la fibre 11 est un tube de verre ayant un diamètre intérieur de 0,003 m et une longueur de 0,25 m. La position du tube 102 par rapport au bac de coagulation 101 est ajustée de façon que la hauteur d'eau entre l'extrémité supérieure du tube 102 et le niveau de référence soit de 0,005 m. L'extrémité inférieure du tube 102 est placée à 0,05 m du niveau de liquide dans le second bac de coagulation 14.

La hauteur de la filière 7 au dessus du bac de coagulation 101 est réglée de façon que l'orifice de la filière 7 soit à 0,002 m du niveau d'eau de référence.

Le contenu des bacs de coagulation 101, 14 et de lavage 19 est maintenu 25°C.

Les valeurs de consigne pour les paramètres de fonctionnement qui sont communiquées à l'unité de commande 6 sont les suivantes : pression dans le réservoir 1 = pression atmosphérique + 80 mmHg (10664 Pa) ; pression dans le réservoir 2 = pression atmosphérique + 100 mmHg (13330 Pa) ; le temps d'interruption de l'injection de la suspension de cellules dans la filière 7 est fixé à 0,40 seconde toutes les 5,8 secondes.

Dans ces conditions de fonctionnement et avec le matériel décrit plus haut, on a obtenu, après la phase de démarrage, une fibre tubulaire segmentée ayant un diamètre interne d'environ 1050 µm, une paroi d'une épaisseur d'environ 150 µm, et des compartiments internes ayant une longueur d'environ 0,6 m. Un capsule implantable comprenant environ 30 000 IE, formée avec cette fibre tubulaire, a une longueur d'environ 3,2 m (soit cinq compartiments).

Le transfert de la fibre 11 du cylindre 18 sur le mandrin 20 a été effectué une heure après la fabrication de la fibre. Après 18 heures à 37°C, la fibre , désolidarisée du mandrin 20 conserve la forme d'une spirale.

### Exemple 2:

Le réservoir 2 a été rempli d'une solution de polymère contenant 16 % en poids d'un copolymère d'acrylonitrile et d'acétate de vinyle, et 84 % en poids de diméthylformamide (DMF). Le contenu du réservoir 2 est à température ambiante (environ 25°C).

Le réservoir 1 a été rempli d'une solution de Bleu Dextran (Sigma - D5751) à 1% en poids dans du sérum physiologique. Le contenu du réservoir 1 est à température ambiante.

Les bacs de coagulation 101 et 14 sont remplis de liquide physiologique stérile qui est renouvelé en permanence. La filière utilisée est identique à celle qui est utilisée dans l'exemple 1.

Le tube 102 des moyens pour provoquer la solidification de la fibre 11 est un tube de verre ayant un diamètre intérieur de 0,003 m et une longueur de 0,10 m. La position du tube 102 par rapport au bac de coagulation 101 est ajustée de la façon que la hauteur d'eau entre l'extrémité supérieure du tube 102 et le niveau de référence soit de 0,005 m. L'extrémité inférieure du tube 102 est placée à 0,05 m du niveau de liquide dans le second bac de coagulation 14.

La hauteur de la filière 7 au dessus du bac de coagulation 101 est réglée de façon que l'orifice de la filière 7 soit à 0,002 m du niveau d'eau de référence.

Le contenu des bacs de coagulation 101, 14 et de lavage 19 est maintenu à 25°C.

Les valeurs de consigne pour les paramètres de fonctionnement qui sont communiquées à l'unité de commande 6 sont les suivantes : pression dans le réservoir 1 = pression atmosphérique + 39 mmHg (5199 Pa), pression dans le réservoir 2 = pression atmosphérique + 360 mmHg (47998 Pa) ; le temps d'interruption de l'injection de la solution de Bleu Dextran dans la filière 7 est fixé à 0,75 seconde toutes les secondes.

Dans ces conditions de fonctionnement et avec le matériel décrit plus haut, on a obtenu, après la phase de démarrage, une fibre .tubulaire segmentée ayant un diamètre interne d'environ 980 µm, une paroi d'une épaisseur d'environ 180 µm et des compartiments internes ayant une longueur d'environ 0,05 m.

Le transfert de la fibre 11 du cylindre 18 sur le mandrin 20 a été effectué une heure après la fabrication de la fibre. Après 24 heures à température ambiante, la fibre désolidarisée du mandrin 20 conserve la forme d'une spirale.

### Exemple 3:

Le réservoir 2 a été rempli d'une solution de polymère contenant 15 % en poids de polyéthersulfone (PES), 5 % en poids de polyéthylèneoxide de masse moléculaire égale à 10 k Dalton (Sigma-P6667), et 80 % en poids de N - méthylpyrrolidone (NMP). Le contenu du réservoir 2 est à température ambiante (environ 25°C).

Le réservoir 1 a été rempli d'une solution de Bleu Dextran (Sigma - D5751) à 1% en poids dans du sérum physiologique. Le contenu du réservoir est à température ambiante.

Les bacs de coagulation 101 et 14 sont remplis de liquide physiologique stérile qui est renouvelé en permanence. La filière utilisée est identique à celle qui est utilisée dans l'exemple 1,

Le tube 102 des moyens pour provoquer la solidification de la fibre 11 est un tube de verre ayant un diamètre intérieur de 0,003 m et une longueur de 0,10 m. La position du tube 102 par rapport au bac de coagulation 101 est ajustée de la façon que la hauteur d'eau entre l'extrémité supérieure du tube 102 et le niveau de référence soit de 0,005 m. L'extrémité inférieure du tube 102 est placée à 0,05 m du niveau de liquide dans le second bac de coagulation 14.

La hauteur de la filière 7 au dessus du bac de coagulation 101 est réglée de façon que l'orifice de la filière 7 soit à 0,002 m du niveau d'eau de référence.

Le contenu des bacs de coagulation 101, 14 et de lavage 19 est maintenu à 25°C.

Les valeurs de consigne pour les paramètres de fonctionnement qui sont communiquées à l'unité de commande 6 sont les suivantes : pression dans le réservoir 1 = pression atmosphérique + 50 mmHg (6665 Pa), pression dans le réservoir 2 = pression atmosphérique + 110 mmHg (13330 Pa); le temps d'interruption de l'injection de la solution de Bleu Dextran dans la filière 7 a été fixé, lors d'un premier essai, à 0,35 seconde toutes les 1,25 secondes puis, lors d'un deuxième essai, à 0,32 secondes toutes les 0,55 secondes.

Dans ces conditions de fonctionnement et avec le matériel décrit plus haut, on a obtenu, après la phase de démarrage, une fibre tubulaire segmentée ayant un diamètre interne d'environ 900 µm, une paroi d'une épaisseur d'environ 100 µm et des compartiments internes ayant une longueur d'environ 0,08 m lors du premier essai, et 0,03 m lors du deuxième essai.

Le transfert de la fibre 11 du cylindre 18 sur le mandrin 20 a été effectué une heure après la fabrication de la fibre. Après 24 heures à température ambiante, la fibre désolidarisée du mandrin 20 conserve la forme d'une spirale.

L'invention n'est pas limitée au mode de réalisation qui vient d'être décrit.

## Revendications

1. Procédé de fabrication d'une capsule tubulaire comprenant une fibre (11 ) ayant une paroi délimitant des compartiments (11 b) internes isolés les uns des autres, la paroi étant faite à partir d'une solution d'au moins un polymère et les compartiments (11b) étant remplis d'un milieu biologiquement actif, le procédé comprenant les étapes de:
- coextruder la solution d'au moins un polymère et le milieu biologiquement actif par injection simultanée de la solution de polymère et du milieu biologiquement actif dans une filière (7) ayant des dimensions déterminées,
- interrompre à intervalles de temps déterminés l'injection du milieu biologiquement actif pour ménager dans la fibre (11 ) des compartiments (11 b) successifs remplis par le milieu biologiquement actif et séparés par un tronçon (11a) intercalaire plein constitué uniquement de la solution de polymère,
- achever la coagulation de la fibre (11 ),
le procédé étant **caractérisé en ce qu'**il comporte l'étape de:
- provoquer, au sortir de la filière (7) et avant l'achèvement de la coagulation, une solidification précoce partielle de la fibre (11) suffisante pour prévenir la rupture de la fibre (11) au niveau des tronçons intercalaires (11a).

2. Procédé selon la revendication 1, **caractérisé en ce que** l'étape de solidification précoce partielle et l'étape d'achèvement de coagulation sont obtenues par immersion dans un liquide de coagulation.

3. Procédé selon la revendication 2, **caractérisé en ce que** l'étape de solidification précoce partielle de la fibre (11 ) comprend les étapes de:
- immerger la fibre (11 ) dans un liquide de coagulation au sortir de la filière (7) de façon à initier une coagulation précoce de la solution de polymère par l'extérieur de la fibre (11); et
- entraîner simultanément la fibre (11) dans le liquide de coagulation selon un trajet déterminé.

4. Procédé selon la revendication 1 ou 2 ou 3, **caractérisé en ce qu'**il comporte en outre, simultanément à l'étape de solidification partielle de la fibre (11), l'étape d'exercer une force de traction déterminée sur la fibre (11 ) de façon à lui conférer au moins une caractéristique géométrique indépendante des dimensions de la filière (7).

5. Procédé selon une des revendications 2 ou 3 ou 4, **caractérisé en ce que** la fibre (11) est maintenue immergée dans un liquide de coagulation jusqu'à être suffisamment solidifiée pour être manipulable.

6. Procédé selon une des revendications 2 à 5, **caractérisé en ce qu'**il comporte en outre l'étape de laver la fibre (11) avec un liquide de lavage après sa solidification dans le liquide de coagulation.

7. Procédé selon une des revendications 1 à 6, **caractérisé en ce que** la température du liquide de coagulation et/ou la température du liquide de lavage sont maintenues à température constante.

8. Procédé selon une des revendications 1 à 7, **caractérisé en ce qu'**il comporte en outre l'étape de donner à la fibre (11) une forme permanente.

9. Procédé selon la revendication 8, **caractérisé en ce que** l'étape de donner à la fibre (11) une forme permanente consiste à enrouler la fibre (11) sur un mandrin (20) cylindrique de façon à lui donner la forme d'une spirale.

10. Procédé selon la revendication 9, **caractérisé en ce que** le pas de la spirale est choisi de façon que chaque portion de fibre (11) comprenant un nombre entier de compartiments (11 b) occupe une longueur de mandrin (20) déterminée.

11. Procédé selon une des revendications 2 à 10, **caractérisé en ce que** le liquide de coagulation est renouvelé en permanence.

12. Procédé selon une des revendications 1 à 11, **caractérisé en ce que** la solution d'au moins un polymère comprend un copolymère d'acrylonitrile et de méthallyle sulfonate de sodium, de l'eau et du dimethylsulfoxide.

13. Procédé selon une des revendications 1 à 12, **caractérisé en ce que** le milieu biologiquement actif est une suspension de cellules.

14. Dispositif de fabrication d'une fibre (11) tubulaire comprenant une paroi délimitant des compartiments (11b) internes isolés les uns des autres et remplis d'un milieu biologiquement actif, comprenant:
- des moyens de filage (7) ayant des dimensions déterminées pour coextruder une solution d'au moins un polymère et le milieu biologiquement actif de façon à obtenir une fibre (11) tubulaire ayant une paroi faite à partir de la solution de polymère et remplie du milieu biologiquement actif;
- des moyens d'alimentation (1, 3, 5) des moyens de filage (7) avec la solution de polymère et avec le milieu biologiquement actif;
- des moyens (6) pour commander l'alimentation simultanée des moyens de filage (7) en solution de polymère et en milieu biologiquement actif et pour interrompre l'alimentation en milieu biologiquement actif à intervalles de temps déterminés de façon à ménager dans la fibre (11 ) des compartiments (11b) successifs remplis par le milieu biologiquement actif et séparés par un tronçon (11a) intercalaire plein constitué uniquement par la solution de polymère;
- des moyens (14) pour achever la coagulation de la fibre (11);
**caractérisé en ce qu'**il comporte:
- des moyens (10) pour provoquer une solidification précoce partielle de la fibre (11) au sortir des moyens de filage (7), cette solidification étant suffisante pour prévenir la rupture de la fibre (11) au niveau des tronçons (11a) intercalaires
- lesdits moyens (10) étant placés en amont des moyens (14) pour achever la coagulation de la fibre dans le sens de déplacement de la fibre.

15. Dispositif de fabrication selon la revendication 14, **caractérisé en ce que** les moyens (10) de solidification précoce partielle comprennent un bac (101) pour liquide de coagulation et les moyens (14) pour achever la coagulation comprennent un second bac de coagulation.

16. Dispositif selon la revendication 14 ou 15, **caractérisé en ce que** les moyens pour provoquer une solidification partielle précoce de la fibre (11) comprennent :
- un bac (101) pour un liquide de coagulation disposé sous les moyens de filage (7) à une distance choisie pour qu'une fibre (11) s'écoulant de la filière à un débit donné commence à coaguler précocement de l'extérieur et ne se rompe pas; et
- des moyens (101, 102) pour entraîner la fibre (11) dans le liquide de coagulation selon un trajet déterminé.

17. Dispositif selon une des revendications 14 à 16, **caractérisé en ce qu'**il comprend en outre des moyens (101, 102) pour exercer une force de traction déterminée sur la fibre (11) de façon à lui conférer au moins une caractéristique géométrique indépendante des dimensions des moyens de filage (7).

18. Dispositif selon la revendication 16 ou 17, **caractérisé en ce que** les moyens d'entraînement et les moyens pour exercer une force de traction comprennent un tube (102) traversant le fond du bac (101) de coagulation et ayant une extrémité supérieure à l'intérieur du bac (101) et une extrémité inférieure à l'extérieur du bac (101), ce tube (102) étant disposé sensiblement à la verticale et son extrémité supérieure étant située au dessous de la surface du liquide de coagulation lorsque le bac (101) est rempli à un niveau de référence.

19. Dispositif selon une des revendications 15 à 18, **caractérisé en ce qu'**il comprend en outre des moyens (12, 13, 15, 103,104) pour renouveler en continu le liquide de coagulation.

20. Dispositif selon une des revendications 14 à 18, **caractérisé en ce qu'**il comprend en outre un bac de lavage (19) pour laver la fibre (11).

21. Dispositif selon une des revendications 14 à 19, **caractérisé en ce qu'**il comprend en outre des moyens pour réguler la température du liquide de coagulation et/ou du liquide de lavage.

22. Dispositif selon une des revendications 14 à 21, **caractérisé en ce qu'**il comprend en outre des moyens (20, 23, 29, 30, 31) pour donner à la fibre (11) une forme déterminée.

23. Dispositif selon la revendications 22, **caractérisé en ce que** la forme déterminée est une spirale et que les moyens pour donner à la fibre (11) cette forme comprennent:
- un mandrin (20);
- des moyens (23) pour entraîner le mandrin (20) en rotation;
- un dispositif de trancanage (29, 30, 31) pour déplacer la fibre (11) selon un mouvement de va-et-vient parallèlement au mandrin (20).

24. Dispositif selon la revendications 23, **caractérisé en ce que** le mandrin (20) est constitué de portions démontables (21).

25. Dispositif selon la revendications 23, **caractérisé en ce que**, en fonctionnement, la vitesse de rotation du mandrin (20) et la vitesse du mouvement de va-et-vient du dispositif de trancanage (29, 30, 31) sont choisies pour que la section de fibre (11 ) enroulée en spirale sur la longueur d'une portion (21) de mandrin (20) corresponde à un nombre entier déterminé de compartiments (11 b) internes de la fibre (11).

## Claims

1. Method for making a tubular capsule comprising a fiber (11) having a wall delimiting inner sections (11b) isolated from one another, said wall being obtained from a solution of at least a polymer and said sections (11b) being filled with a biologically active medium, said method comprising the following steps:
- coextruding the solution of at least a polymer and the biologically active medium by simultaneous injection of the polymer solution and of the biologically active medium into a spinneret (7) with a given size,
- interrupt at given time intervals the injection of the biologically active medium so as to insert into the fiber (11) consecutive sections (11b) filled with the biologically active medium and separated by an intermediate full section (11a) made only by the polymer solution,
- carry out fiber (11) coagulation,
said method being **characterized in that** it comprises the following step:
- start at spinneret (7) exit and before carrying out coagulation an early partial solidification of the fiber (11) sufficient to prevent the fiber (11) from breaking on intermediate sections (lla).

2. Method according to claim 1, **characterized in that** the early partial solidification step and the coagulation step are obtained by immersion in a coagulation liquid.

3. Method according to claim 2, **characterized in that** the step of early partial solidification of the fiber (11) comprises the following steps:
- immerse the fiber (11) in a coagulation liquid at spinneret (7) exit so as to start an early coagulation of the polymer solution through the outside part of the fiber (11); and
- simultaneously drive the fiber (11) in the coagulation liquid along a given path.

4. Method according to claim 1 or 2 or 3, **characterized in that** it further comprises, simultaneously to the step of partial solidification of the fiber (11), a step in which a given traction force is exerted onto the fiber (11) so as to give the latter at least a geometrical feature not depending on spinneret (7) size.

5. Method according to one of claims 2 or 3 or 4, **characterized in that** the fiber (11) is kept immersed in a coagulation liquid until it is sufficiently solidified to be handled.

6. Method according to one of claims 2 to 5, **characterized in that** it further comprises the step in which the fiber (11) is washed with a washing liquid after solidification in the coagulation liquid.

7. Method according to one of claims 1 to 6, **characterized in that** the temperature of the coagulation liquid and/or the temperature of the washing liquid are kept constant.

8. Method according to one of claims 1 to 7, **characterized in that** it further comprises the step in which the fiber (11) is given a permanent shape.

9. Method according to claim 8, **characterized in that** the step in which the fiber (11) is given a permanent shape consists in rolling up the fiber (11) on a cylindrical spindle (20) so as to give it a spiral shape.

10. Method according to claim 9, **characterized in that** the spiral pitch is chosen so that each fiber (11) portion comprising a whole number of sections (11) occupies a given spindle (20) length.

11. Method according to one of claims 2 to 10, **characterized in that** the coagulation liquid is continuously renewed.

12. Method according to one of claims 1 to 11, **characterized in that** the solution of at least a polymer comprises an acrylonitrile-sodium methallyl sulfonate copolymer, water and dimethyl sulfoxid.

13. Method according to one of claims 1 to 12, **characterized in that** the biologically active medium is a cell suspension.

14. Device for making a tubular fiber (11) comprising a wall delimiting inner sections (11b) isolated from one another and filled with a biologically active medium, comprising:
- spinning means (7) having a given size for coextruding a solution of at least a polymer and the biologically active medium so as to obtain a tubular fiber (11) having a wall obtained starting from the polymer solution and filled with the biologically active medium;
- supplying means (1, 3, 5) for supplying the spinning means (7) with the polymer solution and with the biologically active medium;
- means (6) for controlling the simultaneous supply of the spinning means (7) with polymer solution and with biologically active medium et for interrupting the supply with biologically active medium at given time intervals so as to insert into the fiber (11) consecutive sections (11b) filled with the biologically active medium and separated by an intermediate full section (11a) made only by the polymer solution;
- means (14) for coagulating the fiber (11);
**characterized in that** it comprises:
- means (10) for obtaining an early partial solidification of the fiber (11) at the exit of the spinning means (7), said solidification being sufficient to prevent the fiber (11) from breaking on the intermediate sections (11a)
- said means (10) being arranged upstream from the means (14) for coagulating the fiber in fiber shifting direction.

15. Manufacturing device according to claim 14, **characterized in that** the early partial solidification means (10) comprise a vessel (101) for the coagulation liquid and the coagulation means (14) comprise a second coagulation vessel.

16. Device according to claim 14 or 15, **characterized in that** the means for obtaining an early partial solidification of the fiber (11) comprise:
- a vessel (101) for a coagulation liquid arranged under the spinning means (7) at a distance chosen so that a fiber (11) flowing from the spinneret at a given flow rate starts coagulating early from outside and does not break;
- means (101, 102) for driving the fiber (11) in the coagulation liquid along a given path.

17. Device according to one of claims 14 to 16, **characterized in that** it further comprises means (101, 102) for exerting a given traction force onto the fiber (11) so as to give the latter a geometrical feature not depending on the size of the spinning means (7).

18. Device according to claim 16 or 17, **characterized in that** the driving means and the means for exerting a traction force comprise a tube (102) crossing the bottom of the coagulation vessel (101) and having an upper end inside the vessel (101) and a lower end outside the vessel (101), said tube (102) being arranged nearly vertical and its upper end being placed over the surface of the coagulation liquid when the vessel (101) is filled to a reference level.

19. Device according to one of claims 15 to 18, **characterized in that** it further comprises means (12, 13, 15, 103, 104) for continuously renewing the coagulation liquid.

20. Device according to one of claims 14 to 18, **characterized in that** it further comprises a washing vessel (19) for washing the fiber (11).

21. Device according to one of claims 14 to 18, **characterized in that** it further comprises means for regulating the temperature of the coagulation liquid and/or of the washing liquid.

22. Device according to one of claims 14 to 21, **characterized in that** it further comprises means (20, 23, 29, 30, 31) for giving the fiber (11) a given shape.

23. Device according to claim 22, **characterized in that** the given shape is a spiral and **in that** the means for giving the fiber (11) said shape comprise:
- a spindle (20);
- means (23) for turning the spindle (20);
- a guiding device (29, 30, 31) for shifting the fiber (11) according to a forward-backward movement parallel to the spindle (20).

24. Device according to claim 23, **characterized in that** the spindle (20) is made of dismountable portions (21).

25. Device according to claim 23, **characterized in that** during operation the rotation speed of the spindle (20) and the speed of the forward-backward movement of the guiding device (29, 30, 31) are chosen so that the fiber (11) section rolled up as a spiral on the length of a spindle (20) portion (21) corresponds to a given whole number of inner sections (11b) of the fiber (11).

## Patentansprüche

1. Verfahren zur Herstellung von einer eine Fiber (11) umfassenden rohrförmigen Kapsel, die eine voneinander isolierte innere Abteile (11b) abgrenzende Wand aufweist, wobei die Wand ausgehend von einer Lösung aus mindestens einem Polymer hergestellt ist und die Abteile (11b) mit einer biologisch aktiven Zusammensetzung gefüllt sind, wobei das Verfahren die folgenden Schritte umfasst:
- Koextrusion der Lösung aus mindestens einem Polymer und der biologisch aktiven Zusammensetzung durch gleichzeitiges Einspritzen der Polymerlösung und der biologisch aktiven Zusammensetzung in eine Spinndüse (7) vorgegebener Größe,
- Unterbrechung bei vorgegebenen Zeitintervallen des Einspritzens von der biologisch aktiven Zusammensetzung zur Einführung in die Fiber (11) von aneinanderfolgenden Abteilen (11b), die mit der biologisch aktiven Zusammensetzung gefüllt sind und durch einen zwischengeschalteten Vollabschnitt (11a) aus nur der Polymerlösung getrennt sind,
- Koagulation der Fiber (11),
wobei das Verfahren **dadurch gekennzeichnet ist, daß** es den folgenden Schritt umfasst:
- Auslösung am Ausgang der Spinndüse (7) und vor der Koagulationsbeendung einer vorzeitigen Teilerstarrung der Fiber (11), die zum Verhindern einer Brechung der Fiber (11) an den zwischengeschalteten Abteilen (11a) genügt.

2. Verfahren nach Anspruch 1, **dadurch gekennzeichnet, daß** der Schritt von vorzeitiger Teilerstarrung und der Koagulationsschritt durch Eintauchen in eine Koagulationsflüssigkeit erhalten werden.

3. Verfahren nach Anspruch 2, **dadurch gekennzeichnet, daß** der Schritt von vorzeitiger Teilerstarrung der Fiber (11) die folgenden Schritte umfasst:
- Eintauchen der Fiber (11) in eine Koagulationsflüssigkeit am Ausgang der Spinndüse (7), um eine vorzeitige Koagulation der Polymerlösung durch die Außenseite der Fiber (11) zu starten; und
- gleichzeitiges Führen der Fiber (11) in der Koagulationsflüssigkeit nach einem vorgegebenen Weg.

4. Verfahren nach Anspruch 1 oder 2 oder 3, **dadurch gekennzeichnet, daß** es gleichzeitig zum Schritt von Teilerstarrung der Fiber (11) den Schritt weiter umfasst, in dem eine vorgegebene Zugkraft auf die Fiber (11) ausgeübt wird, um derselbe mindestens eine von der Größe der Spinndüse (7) unabhängige geometrische Eigenschaft zu verleihen.

5. Verfahren nach einem der Ansprüche 2 oder 3 oder 4, **dadurch gekennzeichnet, daß** die Fiber (11) in eine Koagulationsflüssigkeit getaucht bleibt, bis sie zur Handhabung genug erstarrt ist.

6. Verfahren nach einem der Ansprüche 2 bis 5, **dadurch gekennzeichnet, daß** es den Schritt weiter umfasst, in dem die Fiber (11) nach ihrer Erstarrung in der Koagulationsflüssigkeit mit einer Spülflüssigkeit gewaschen wird.

7. Verfahren nach einem der Ansprüche 1 bis 6, **dadurch gekennzeichnet, daß** die Temperatur der Koagulationsflüssigkeit und/oder die Temperatur der Spülflüssigkeit konstant gehalten werden.

8. Verfahren nach einem der Ansprüche 1 bis 7, **dadurch gekennzeichnet, daß** es den Schritt weiter umfasst, in dem der Fiber (11) eine permanente Gestalt verleiht wird.

9. Verfahren nach Anspruch 8, **dadurch gekennzeichnet, daß** der Schritt, in dem der Fiber (11) eine permanente Gestalt verleiht wird, die Aufwicklung der Fiber (11) auf einer zylindrischen Spindel (20) umfasst, um ihr eine Spiralegestalt zu verleihen.

10. Verfahren nach Anspruch 9, **dadurch gekennzeichnet, daß** die Spiralesteigung so ausgewählt ist, daß jeder Fiberabschnitt (11) umfassend eine ganze Zahl von Abteilen (11b) eine vorgegebene Länge der Spindel (20) besetzt.

11. Verfahren nach einem der Ansprüche 2 bis 10, **dadurch gekennzeichnet, daß** die Koagulationsflüssigkeit konstant erneut wird.

12. Verfahren nach einem der Ansprüche 1 bis 11, **dadurch gekennzeichnet, daß** die Lösung aus mindestens einem Polymer ein Acrylnitryl/Natrium-Methallyl-Sulfonat-Copolymer, Wasser und Dimethylsulfoxyd enthält.

13. Verfahren nach einem der Ansprüche 1 bis 12, **dadurch gekennzeichnet, daß** die biologisch aktive Zusammensetzung eine Zellsuspension ist.

14. Vorrichtung zur Herstellung einer rohrförmigen Fiber (11), die eine voneinander isolierte und mit einer biologisch aktiven Zusammensetzung gefüllte innere Abteile (11b) abgrenzende Wand aufweist, umfassend:
- Spinnenmittel (7) vorgegebener Größe zur Koextrusion einer Lösung aus mindestens einem Polymer und der biologisch aktiven Zusammensetzung, um eine rohrförmige Fiber (11) mit einer Wand zu erhalten, die ausgehend von der Polymerlösung hergestellt und mit der biologisch aktiven Zusammensetzung gefüllt ist;
- Versorgungsmittel (1, 3, 5) der Spinnenmittel (7) mit der Polymerlösung und mit der biologisch aktiven Zusammensetzung;
- Mittel (6) zur Steuerung der gleichzeitigen Versorgung der Spinnenmittel (7) mit der Polymerlösung und mit der biologisch aktiven Zusammensetzung und zur Unterbrechung der Versorgung mit der biologisch aktiven Zusammensetzung bei vorgegebenen Zeitintervallen zur Einführung in die Fiber (11) von aneinanderfolgenden Abteilen (11b), die mit der biologisch aktiven Zusammensetzung gefüllt sind und durch einen zwischengeschalteten Vollabschnitt (11a) aus nur der Polymerlösung getrennt sind;
- Mittel (14) zur Koagulation der Fiber (11);
**dadurch gekennzeichnet, daß** sie:
- Mittel (10) zur Auslösung einer vorzeitigen Teilerstarrung der Fiber (11) am Ausgang der Spinnenmittel (7) umfasst, wobei diese Erstarrung zum Verhindern einer Brechung der Fiber (11) an den zwischengeschalteten Abteilen (11a) genügt
- wobei diese Mittel (10) stromauf der Mittel (14) zur Koagulation der Fiber in Fiberbewegungsrichtung angeordnet sind.

15. Herstellungsvorrichtung nach Anspruch 14, **dadurch gekennzeichnet, daß** die Mittel (10) zur vorzeitigen Teilerstarrung einen Behälter (101) für die Koagulationsflüssigkeit umfassen und die Koagulationsmittel (14) einen zweiten Koagulationsbehälter umfassen.

16. Vorrichtung nach Anspruch 14 oder 15, **dadurch gekennzeichnet, daß** die Mittel zur Auslösung einer vorzeitigen Teilerstarrung der Fiber (11) die folgenden Elemente umfassen:
- einen Behälter (101) für eine Koagulationsflüssigkeit, der unter den Spinnenmitteln (7) angeordnet sind, mit einem Abstand, der so ausgewählt ist, daß eine aus der Spinndüse mit einem vorgegebenen Durchfluss ausgeflossenen Fiber (11) vorzeitig aus der Außenseite ihre Koagulation beginnt und sich nicht bricht; und
- Mittel (101, 102) zum Führen der Fiber (11) in der Koagulationsflüssigkeit nach einem vorgegebenen Weg.

17. Vorrichtung nach einem der Ansprüche 14 bis 16, **dadurch gekennzeichnet, daß** sie Mittel (101, 102) zur Ausübung einer vorgegebenen Zugkraft auf die Fiber (11) umfasst, um derselbe mindestens eine von der Größe der Spinnenmittel (7) unabhängige geometrische Eigenschaft zu verleihen.

18. Vorrichtung nach Anspruch 16 oder 17, **dadurch gekennzeichnet, daß** die Führungsmittel und die Mittel zur Ausübung einer Zugkraft ein den Boden des Koagulationsbehälters (101) durchgehendes Rohr (102) umfasst, das ein oberes Ende innerhalb des Behälters (101) und ein unteres Ende außerhalb des Behälters (101) aufweist, wobei dieses Rohr (102) deutlich vertikal angeordnet ist und dessen obere Ende unter der Oberfläche der Koagulationsflüssigkeit liegt, wenn der Behälter (101) zum Bezugsniveau gefüllt ist.

19. Vorrichtung nach einem der Ansprüche 15 bis 18, **dadurch gekennzeichnet, daß** sie Mittel (12, 13, 15, 103, 104) zum konstanten Erneuern der Koagulationsflüssigkeit weiter umfasst.

20. Vorrichtung nach einem der Ansprüche 14 bis 18, **dadurch gekennzeichnet, daß** sie einen Spülbehälter (19) zum Waschen der Fiber (11) weiter umfasst.

21. Vorrichtung nach einem der Ansprüche 14 bis 19, **dadurch gekennzeichnet, daß** sie Mittel zum Einstellen der Temperatur der Koagulationsflüssigkeit und/oder der Spülflüssigkeit weiter umfasst.

22. Vorrichtung nach einem der Ansprüche 14 bis 21, **dadurch gekennzeichnet, daß** sie Mittel (20, 23, 29, 30, 31) zum Verleihen zu der Fiber (11) einer vorgegebenen Gestalt weiter umfasst.

23. Vorrichtung nach Anspruch 22, **dadurch gekennzeichnet, daß** die vorgegebene Gestalt eine Spirale ist, und daß die Mittel zum Verleihen zu der Fiber (11) dieser Gestalt:
- eine Spindel (20);
- Mittel (23) zum Drehen der Spindel (20);
- eine Führungseinrichtung (29, 30, 31) zum Verschieben der Fiber (11) nach einer Vorwärts-Rückwärts-Bewegung parallel zur Spindel (20), umfassen.

24. Vorrichtung nach Anspruch 23, **dadurch gekennzeichnet, daß** die Spindel (20) aus demontierbaren Abschnitten (21) besteht.

25. Vorrichtung nach Anspruch 23, **dadurch gekennzeichnet, daß** bei dem Betrieb die Drehgeschwindigkeit der Spindel (20) und die Geschwindigkeit der Vorwärts-Rückwärts-Bewegung der Führungseinrichtung (29, 30, 31) so ausgewählt sind, daß der auf der Länge eines Abschnitts (21) der Spindel (20) spiralabgewickelte Fiberschnitt (11) einer vorgegebenen ganzen Zahl von inneren Abteilen (11b) der Fiber (11) entspricht.
